# EUROPEAN PATENT SPECIFICATION

(11) **EP 2 799 869 B1**
(45) Date of publication and mention of the grant of the patent: **08.02.2017**
(21) Application number: 14166180.1
(22) Date of filing: 28.04.2014
(51) Int. Cl.: G01N 33/487

(54) **Apparatus and method for reading identification information of biosensor**
Vorrichtung und Verfahren zum Lesen von Identifizierungsinformationen eines Biosensors
Appareil et procédé pour lire des informations d'identification de biocapteur

(30) Priority: 29.04.2013 KR 20130047617
(43) Date of publication of application: 05.11.2014
(73) Proprietor: Infopia Co., Ltd., Gyeonggi-do (KR)
(72) Inventor: BAE, Byeong-Woo, Gyeonggi-do (KR); SUK, Hong-Seong, Gyeonggi-do (KR); KIM, Gu-Youn, Gyeonggi-do (KR); JEONG, Chan-Kwan, Gyeonggi-do (KR); SEO, Young-Jun, Gyeonggi-do (KR); HONG, Ju-Pyo, Gyeonggi-do (KR)
(74) Representative: Gill, Siân Victoria

(56) References cited:
- WO-A2-2010/016277
- US-A1- 2007 108 392
- US-A1- 2008 275 365
- US-A1- 2012 068 629

## Description

### BACKGROUND

### 1. Field

The following description relates to a technology for measuring biometric data, and more specifically, to a technology for reading identification information recorded depending on a color on a surface of the biosensor.

### 2. Description of the Related Art

There are some devices for measuring biometric data using a disposable biosensor. Here, the biosensor, e.g. a strip of a blood glucose measuring device, may have different characteristics for each product lot number. Also, in the existing devices, correction code information for correcting such characteristics is recorded on a surface of the biosensor depending on the arrangement of colors. The existing device for measuring biometric data, which reads the arrangement of colors, disposes light sources and light detectors at a one-to-one ratio for each segment that makes arrangement patterns. Such existing devices have problems of complicating a circuit and increasing its cost of production, among others.

Korean Patent No. 10-1224600 (filed on May 9, 2011, and registered on January 15, 2013) discloses a bio-chip detector. However, this application uses motors to detect information of a plurality of bio-chips (biosensors) and includes a rotation axis and a board, which are both disposed, thus resulting in the large scale and difficulty to carry it around.

US patent application US 2008/0275365 discloses an apparatus to read identification information of single test strip containers presenting multiple columns of dark/light patches on their surface. Said apparatus comprises multiple pairs of light emitters and receivers that must be read simultaneously during the withdrawal of the container from the reading apparatus.

### SUMMARY

In reading identification information through a pattern of the existing biosensor, a photodetector and a light resource are required for each segment so as to read the segments of each pattern. Such a system has not only caused a complexity of driving circuits, but it has also raised manufacturing costs, and prolonged operating hours.

The following description relates to a technology for reading biosensor identification information which, in contrast to the existing technology, uses a smaller amount of photodetectors than light resources, thus simplifying driver circuits and decreasing the amount of work and time needed.

In one general aspect, an apparatus configured to detect an insertion of a biosensor and to read identification of the biosensor is provided, the identification information being recorded according to a pattern of a plurality of color segments on a surface of the biosensor. The apparatus comprises: a light receiver; a plurality of light emitters and an identification code reader, characterised in that the plurality of light emitters are configured to be located corresponding to and facing a plurality of color segments on a surface of the biosensor when the biosensor is inserted into the apparatus, and the plurality of light emitters are arranged around the light receiver; and the identification code reader is configured to sequentially turn on/off the plurality of light emitters according to each position thereof and read the light receiver to read identification information recorded on the segment of the surface of the biosensor which corresponds to and faces a position of the light emitter which has been turned on.

The light receiver may include a plurality of photodetectors, each of which is connected in series or in parallel to each other, and which are capable of being simultaneously operated in entirety thereof by the identification code reader.

The plurality of light emitters may include a plurality of light emitting elements, which are simultaneously operated by the identification code reader.

The identification code reader may be configured to turn on/off the plurality of light emitters by alternating between groups of light emitters arranged as linear arrays along perpendicular directions defined on the plane of the biosensor.

The apparatus may include a plurality of sets of reader modules including the light receiver, the plurality of light emitters, and the identification code reader.

The identification code reader may include: a light emitter driver connected to each of the plurality of light emitters; a light receiver driver configured to drive the light receiver; a converter configured to convert an output of the light receiver to a digital format; and a pattern reader configured to read a pattern of an identification information code of the biosensor from an output of the converter.

In another general aspect, a method for reading identification information of a biosensor is provided, wherein the identification information is recorded according to an arrangement of different colors on a plurality of segments on a surface of the biosensor, the method comprising: a) detecting an insertion of the biosensor; b) turning on one of a plurality of light emitters arranged in a position corresponding to and facing color segments on the surface of the biosensor which has been inserted while turning off the rest; c) detecting, at a light receiver, a light which has been projected from the light emitter and reflected from or penetrated through the biosensor; characterized in that the method further comprises: d) reading, through the detected light, a code recorded in a position corresponding to the plurality of color segments on the surface of the biosensor which has been inserted; and e) reading identification information by repeating operations b) to d) with respect to the plurality of light emitters located in different positions, and by reading the colors of all the segments.

The operation e) may include turning on/off the plurality of light emitters by alternating between groups of light emitters arranged as linear arrays along perpendicular directions defined on the plane of the biosensor.

Other features and aspects may be apparent from the following detailed description, the drawings, and the claims.

### BRIEF DESCRIPTION OF THE DRAWINGS

FIG. 1 is a diagram illustrating an example of an apparatus of reading identification information according to an exemplary embodiment.
FIG. 2 is a diagram illustrating an example of an identification code reader according to an exemplary embodiment.
FIG. 3 is a diagram illustrating an example of an operation of an apparatus of reading identification information according to an exemplary embodiment.
FIG. 4 is a diagram illustrating another example of an operation of an apparatus of reading identification information according to an exemplary embodiment.
FIG. 5 is a diagram illustrating yet another example of an operation of an apparatus of reading identification information according to an exemplary embodiment.
FIG. 6 is a schematic diagram illustrating an example of an apparatus of reading identification information according to an exemplary embodiment.
FIG. 7 is another schematic diagram illustrating an example of an apparatus of reading identification information according to an exemplary embodiment.
FIG. 8 is a flowchart illustrating an example of a method for reading identification information according to an exemplary embodiment.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. Also, descriptions of well-known functions and constructions may be omitted for increased clarity and conciseness.

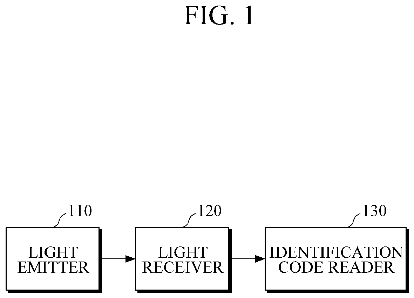

FIG. 1 is a diagram illustrating an example of an apparatus of reading identification information according to an exemplary embodiment.

As illustrated in FIG. 1, the apparatus of reading identification information includes a light receiver 120; a plurality of light emitters 110 whose position corresponds to and faces a plurality of segments on a surface of a biosensor inserted and are disposed around the light receiver 120; and an identification code reader 130 that sequentially turns on/off the plurality of the light emitters 110 according to position thereof and then reads the light receiver 120 to read the color information recorded on the segments which correspond to and face the position of the light emitters 110 which has been turned on.

The light receiver 120 may include a plurality of photodetectors connected in series or in parallel to each other, which are capable of being simultaneously operated in entirety thereof by the identification code reader 130.

In an exemplary embodiment, the light receiver 120 may be a single element. In another exemplary embodiment, the light receiver 120 may include a plurality of light receivers, which are capable of being implemented in one single driving circuit.

The light emitter 110 may include a plurality of light emitting elements, which are capable of being simultaneously operated) by the identification code reader 130. In an exemplary embodiment, a light emitting diode (LED) may be used as the light emitter 110.

The identification code reader 130 may be implemented to turn on/off the plurality of the light emitters 110 by alternating between horizontal and vertical directions

In an exemplary embodiment, the apparatus of reading identification information may include a plurality of sets of reader modules that include a light receiver 120, a plurality of light emitters 110, and an identification code reader 130.

In another exemplary embodiment, the apparatus may include the plurality of reader modules which, in contrast with the existing technology, use the minimum amount of components while being able to accommodate several biosensors.

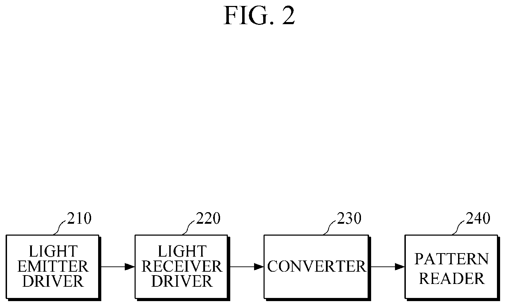

FIG. 2 is a diagram illustrating an example of an identification code reader according to an exemplary embodiment.

An identification code reader 130 may include a light emitter driver 210; a light receiver driver 220 that drives a light receiver; a converter 230 that converts an output of the light receiver into a digital format; and a pattern reader 240 that reads a pattern of an identification information code of a biosensor from the output of the converter 230.

The light emitter driver 210 may drive a light emitter 110 and enable the light emitter 110 to irradiate light on a side where an identification information code is attached.

The light receiver driver 220 may enable a light receiver 120 to detect the light which has been generated by the light emitter 110.

The converter 230 may convert the pattern of the identification information code to a digital format and enable the following pattern reader 240 to analyze the code pattern.

The pattern reader 240 may read information of the pattern, which has been converted to a digital format by the converter 230, and detect the pattern of the identification information code of the biosensor.

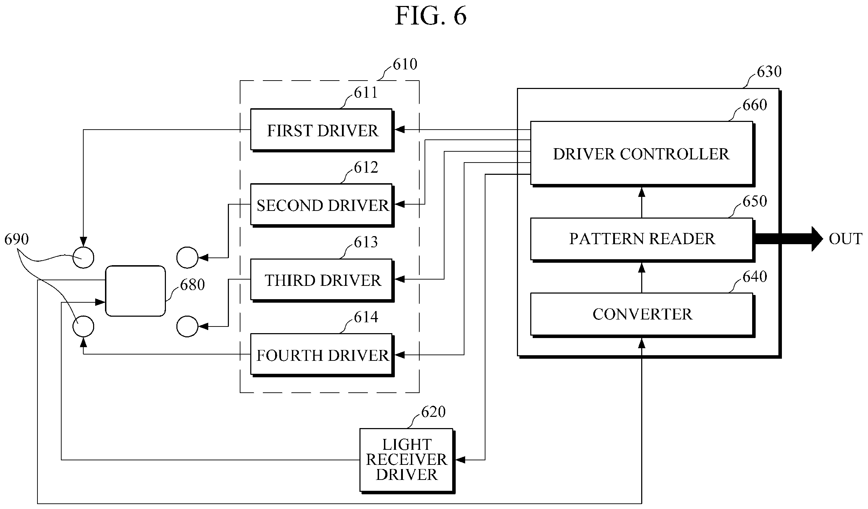

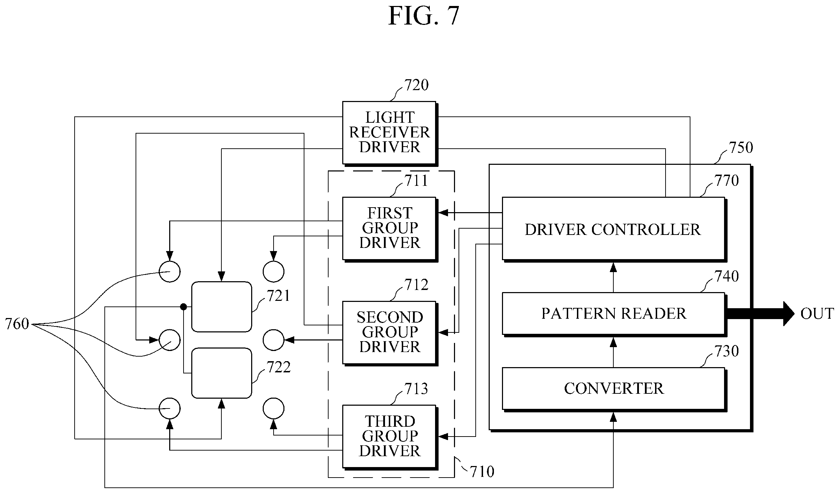

The detailed description about operations of the apparatus according to an exemplary embodiment will be described later in reference to FIGS. 6 and 7.

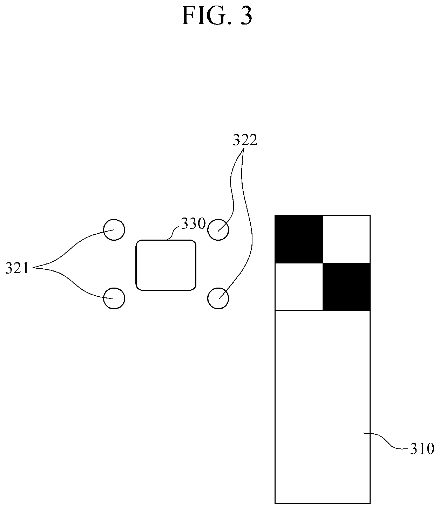

FIG. 3 is a diagram illustrating an example of an operation of an apparatus of reading identification information according to an exemplary embodiment.

In an exemplary embodiment, if a biosensor 310 which includes an identification information code, as illustrated in FIG. 3, is inserted into an apparatus of reading identification information, the apparatus may, for purpose of identifying the identification information code, initially operate the? first light emitters 321 to generate light; operate a light receiver 330; and enable the light receiver 330 to detect the light, which has been generated by the first light emitters 321 and reflected from one side of the biosensor 310. Then, the apparatus may operate second light emitters 322 to generate light, operate the light receiver 330, and enable the light receiver 330 to detect the light reflected from the biosensor 310.

In another exemplary embodiment, the light emitters 321 and 322 may be grouped horizontally, not longitudinally as illustrated in FIG. 3.

The detailed description about operations of the apparatus according to an exemplary embodiment will be described later in reference to FIGS. 6 and 7.

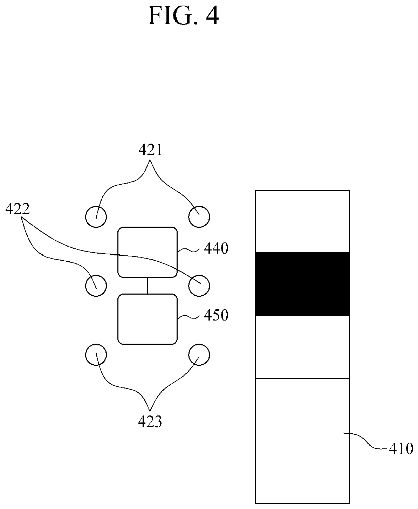

FIG. 4 is a diagram illustrating another example of an operation of an apparatus of reading identification information according to an exemplary embodiment.

In an exemplary embodiment, if a biosensor 410, which includes an identification information code attached thereon as illustrated in FIG. 4, is inserted into an apparatus of reading identification information, the apparatus may, for the purpose of identifying the identification information code, initially operate first light emitters 421 to generate light; operate a light receiver 440; and enable the light receiver 440 to detect the light, which has been generated by the first light emitters 421 and reflected from one side of the biosensor 410. Then, the apparatus may operate second light emitters 422 to generate light; operate light receivers 440 and 450; and enable the light receivers 440 and 450 to detect the light, which has been reflected from the biosensor 410. Then, the apparatus may operate third light emitters 423 to generate light; operate the light receiver 450; and enable the light receiver 450 to detect the light, which has been reflected from the biosensor 410. The light receivers 440 and 450 are electrically connected to each other and may be implemented in one single driving circuit. By these operations, the apparatus may read a pattern of the identification information code, which is included on one side of the biosensor 410.

The detailed description about operations of the apparatus according to an exemplary embodiment will be described later in reference to FIGS. 6 and 7.

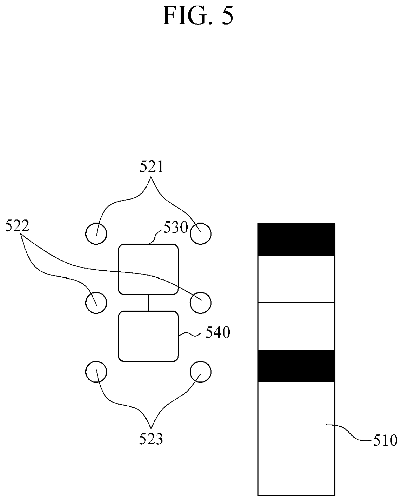

FIG. 5 is a diagram illustrating yet another example of an operation of an apparatus of reading identification information according to an exemplary embodiment.

In an exemplary embodiment, if a biosensor 510, which includes an identification information code attached thereon as illustrated in FIG. 5, is inserted into an apparatus of reading identification information, the apparatus may, for the purpose of identifying the identification information code, initially operate first light emitters 521 to generate light; operate a light receiver 530; and enable the light receiver 530 to detect the light, which has been generated by the first light emitters 521 and reflected from one side of the biosensor 510. Then, the apparatus may operate second light emitters 522 to generate light; operate the light receiver 530; and enable the light receiver 530 to detect the light, which has been reflected from the biosensor 510. Then, the apparatus may re-operate the second light emitters 522 to generate light; operate a light receiver 540; and enable the light receiver 540 to detect the light, which has been reflected from the biosensor 510. At last, the apparatus may operate third light emitters 523 to generate light; operate the light receiver 540; and enable the light receiver 540 to detect the light, which has been reflected from the biosensor 510. The light receivers 530 and 540 are electrically connected to each other and may be implemented in one single driving circuit.

The pattern of the identification information code is not limited to the exemplary embodiments described above in FIGS. 3 to 5. Also, various patterns besides the description above may be attached on one side of the biosensor, and the number of the light emitters or light receivers may be readily changed as necessary.

The detailed description about operations of the apparatus according to an exemplary embodiment will be described later in reference to FIGS. 6 and 7.

FIG. 6 is a schematic diagram illustrating an example of an apparatus of reading identification information according to an exemplary embodiment.

An apparatus of reading identification information is described in detail according to a schematic diagram of circuits as illustrated in FIG. 6. To read a pattern of the biosensor, a driver controller 660 may initially issue an operation instruction to a light emitter driver 610. The light emitter driver 610 may include a first driver 611, a second driver 612, a third driver 613, and a fourth driver 614, each of which controls a light emitter 690. The light emitter driver 610 may drive the first driver 611 and enable the light emitter, which is connected to the first driver 611, to generate the light toward the biosensor. Then, if an instruction is transferred to a light receiver driver 620 by the driver controller 660, the light receiver driver 620 may enable a photodetector of a light receiver 680 to detect the light, which has been reflected from the biosensor. The light information detected from the photodetector of the light receiver 680 may be converted into a digital format by the converter 640, and the converted light information may be input to a pattern reader 650. The pattern reader 650 may read the pattern of the converted light information, which has been input by the converter 640.

Then, the driver controller 660 may request, again through the light emitter driver 610, a light emitter to generate light, wherein the light emitter is connected to the second driver 612, and the light emitter may be operated. The light receiver driver 620 may re-instruct the light receiver 680 to be operated. Light information detected by the light receiver 680 may be transmitted again to the converter 640, which converts again the light information into a digital format and transfers the converted light information to the pattern reader 650.

Then, the driver controller 660 may request, again through the light emitter driver 610, a light emitter to generate light, wherein the light emitter is connected to the third driver 613, and the light emitter may be operated. The driver controller 660 may control the light receiver driver 620 and re-instruct the light receiver 680 to be operated. Light information detected by the light receiver 680 may be re-transmitted to the converter 640, which re-converts the light information into a digital format and transfers the converted light information to the pattern reader 650.

Then, the driver controller 660 may request, again through the light emitter driver 610 , a light emitter to generate light, wherein the light emitter is connected to the fourth driver 614, and the light emitter may be operated. The driver controller 660 may control the light receiver driver 620 and re-instruct the light receiver 680 to be operated. Light information detected by the light receiver 680 may be re-transmitted to the converter 640, which re-converts the light information to a digital format and transfers the converted light information to the pattern reader 650.

By these operations, the pattern reader 650 may read and output the pattern.

FIG. 7 is another schematic diagram illustrating an example of an apparatus of reading identification information according to an exemplary embodiment.

An apparatus of reading identification information is described in detail according to a schematic diagram of circuits as illustrated in FIG. 7. To read a pattern of the biosensor, a light emitter driver 710 may initially issue an operation instruction to light emitters. The light emitter driver 710 may form the light emitters into a group; and may include a first group driver 711, a second group driver 712, and a third group driver 713, which control the grouped light emitters for each group. The light emitter driver 710 may drive the first group driver 711 and enable the light emitters, which are connected to the first group driver 711, to generate light toward the biosensor. Then, after a light receiver driver 720 recognizes the information that the light has been generated, the light receiver driver 720 may enable a photodetector of a light receiver 721 to detect the light that has been reflected from the biosensor. Light information detected by the photodetector of the light receiver 721 may be converted into a digital format by a converter 730, and the converted light information may be input to a pattern reader 740. The pattern reader 740 may read a pattern of the converted light information, which has been input by the converter 730.

Then, the light emitter driver 710 may request each of the light emitters to generate light, wherein the light emitters are connected to the second group driver 712, and the light emitters may be operated. If the light receiver driver 720 receives a signal of the light that has been generated by the light emitters connected to the second group driver 712, the light receiver driver 720 may re-instruct the light receiver 721 to be operated. Light information detected by the light receiver 721 may be re-transmitted to the converter 730, which re-converts the light information into a digital format and transfers the converted light information to the pattern reader 740.

Then, the light emitter driver 710 may request again each of the light emitters to generate light, wherein the light emitters are connected to the second group driver 712, and the light emitters may be operated. If the light receiver driver 720 receives a signal of the light that has been generated by the light emitters connected to the second group driver 712, the light receiver driver 720 may re-instruct a light receiver 722 to be operated. Light information detected by the light receiver 722 may be re-transmitted to the converter 730, which re-converts the light information into a digital format and transfers the converted light information to the pattern reader 740.

Then, the light emitter driver 710 may request each of the light emitters to generate light, wherein the light emitters are connected to the third group driver 713, and the light emitters may be operated. If the light receiver driver 720 receives a signal of the light that has been generated by the light emitters connected to the third group driver, the light receiver driver 720 may re-instruct the light receiver 722 to be operated. Light information detected by the light receiver 722 may be re-transmitted to the converter 730, which re-converts the light information into a digital format and transfers the converted light information to the pattern reader 740.

The light receivers 721 and 722 are electrically connected to each other, and may be implemented in one single driving circuit.

After all of these operations are finished, the apparatus may detect a pattern of an identification information code of a biosensor, and recognize the identification information related to the pattern.

A composition of the apparatus is not limited to the exemplary embodiments above, and may be added or modified as necessary.

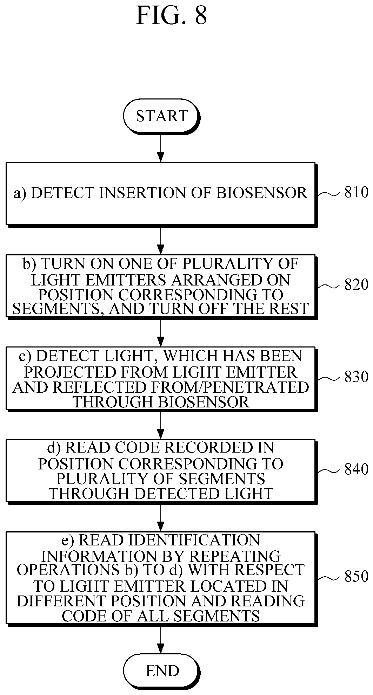

FIG. 8 is a flowchart illustrating an example of a method for reading identification information according to an exemplary embodiment.

As illustrated in FIG. 8, to read identification information recorded according to an arrangement of different colors on a plurality of segments on a surface of a biosensor, a method for reading identification information may include: a) detecting an insertion of a biosensor in 810; b) turning on one of a plurality of light emitters arranged in a position corresponding to and facing segments on the surface of the inserted biosensor, and turning off the rest in 820; c) detecting light, which has been projected from a light emitter and reflected from or penetrated through the biosensor in 830; d) reading a code recorded in a position corresponding to a plurality of segments on the surface of the inserted biosensor through the detected light in 840; and e) reading identification information by repeating operations b) to d) for light emitters located in different positions and reading codes of all segments in 850.

In one exemplary embodiment of the operation e), the method may be implemented to turn on/off a plurality of light emitters by alternating between horizontal and vertical directions.

Compared to the existing technology, the apparatus and method may save resources, reduce the manufacturing costs, and manufacture a small device by using a small amount of read sensors. In addition, driving circuits, particularly in sensing circuits, may be lessened, and several photodetectors are connected in a single circuit resulting in a reduced number of tasks and time needed.

The methods and/or operations described above may be recorded, stored, or fixed in one or more computer-readable storage media that includes program instructions to be implemented by a computer to cause a processor to execute or perform the program instructions. The media may also include, alone or in combination with the program instructions, data files, data structures, and the like. Examples of computer-readable storage media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The described hardware devices may be configured to act as one or more software modules in order to perform the operations and methods described above, or vice versa. In addition, a computer-readable storage medium may be distributed among computer systems connected through a network and computer-readable codes, or program instructions may be stored and executed in a decentralized manner.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. An apparatus configured to detect an insertion of a biosensor and to read identification information of the biosensor (310, 410, 510), the identification information recorded according to a pattern of a plurality of color segments on a surface of the biosensor, and the apparatus comprising
a light receiver (120, 330, 440, 450, 530, 540), a plurality of light emitters (110, 321, 322, 421, 422, 423, 521, 522, 523) located corresponding to and facing a plurality of color segments on a surface of the biosensor (310, 410, 510) when the biosensor (310, 410, 510) is inserted into the apparatus, and an identification code reader (130), **characterized in that** the plurality of light emitters (110, 321, 322, 421, 422, 423, 521, 522, 523) are arranged around the light receiver (120, 330, 440, 450, 530, 540);
and the identification code reader (130) is configured to sequentially turn on/off the plurality of light emitters (110, 321, 322, 421, 422, 423, 521, 522, 523) according to each position thereof, and read the light receiver (120, 330. 440, 450, 530, 540) to read identification information recorded on the segment of the surface of the biosensor which corresponds to and faces a position of the light emitter (110, 321, 322, 421, 422, 423, 521, 522, 523) which has been turned on.

2. The apparatus of claim 1, wherein the light receiver (120, 330, 440, 450, 530, 540) comprises a plurality of photodetectors, which are connected in series or in parallel to each other, and which are capable of being simultaneously operated in entirety thereof by the identification code reader (130).

3. The apparatus of claim 1, wherein the plurality of light emitters (110, 321, 322, 421, 422, 423, 521, 522, 523) comprise a plurality of light emitting elements, which are simultaneously operated by the identification code reader (130).

4. The apparatus of claim 3, wherein the identification code reader (130) is configured to turn on and off the plurality of light emitters (110, 321, 322, 421, 422, 423, 521, 522, 523) by alternating between groups of light emitters arranged as linear arrays along perpendicular directions defined on the plane of the biosensor (310, 410, 510).

5. The apparatus of claim 1, comprising:
a plurality of sets of reader modules including the light receiver (330), the plurality of light emitters (110, 321, 322, 421, 422, 423, 521, 522, 523), and the identification code reader (130).

6. The apparatus of claim 3, wherein the identification code reader (130) comprises:
a light emitter driver (210) connected to each of the plurality of light emitters (110, 321, 322,421,422,423,521,522,523);
a light receiver driver (220) configured to drive the light receiver (120, 330, 440, 450, 530, 540);
a converter (230) configured to convert an output of the light receiver (120, 330, 440, 450, 530, 540) into a digital format; and
a pattern reader (240) configured to read a pattern of the plurality of color segments on a surface of the biosensor (310, 410, 510) from an output of the converter (230).

7. A method for reading identification information of a biosensor (310, 410, 510), wherein the identification information is recorded according to an arrangement of different colors on a plurality of segments on a surface of the biosensor (310, 410, 510), the method comprising:
a) detecting an insertion of the biosensor (310, 410, 510);
b) turning on one of a plurality of light emitters (110, 321, 322, 421, 422, 423, 521, 522, 523) arranged in a position corresponding to and facing color segments on the surface of the biosensor (310, 410, 510) which has been inserted while turning off the rest;
c) detecting, at a light receiver (120, 330, 440, 450, 530, 540), a light, which has been projected from the light emitter (110, 321, 322, 421, 422, 423, 521, 522, 523) and reflected from or penetrated through the biosensor (310, 410, 510); **characterized in that** the method further comprises:
d) reading, through the detected light, a color recorded in a position corresponding to the plurality of color segments on the surface of the biosensor (310, 410, 510) which has been inserted; and
e) reading identification information by repeating operations b) to d) with respect to the plurality of light emitters (110, 321, 322, 421, 422, 423, 521, 522, 523) located in different positions, and by reading the colors of all the segments.

8. The method of claim 7, wherein the operation e) comprises turning on and off the plurality of light emitters (110, 321, 322, 421, 422, 423, 521, 522, 523) by alternating between groups of light emitters arranged as linear arrays along perpendicular directions defined on the plane of the biosensor (310, 410, 510).

## Patentansprüche

1. Vorrichtung, die konfiguriert ist, um einen Einsatz eines Biosensors zu erfassen und um Identifikationsinformationen des Biosensors (310, 410, 510) zu lesen, wobei die Identifikationsinformationen gemäß einem Muster einer Vielzahl von Farbsegmenten an einer Oberfläche des Biosensors aufgezeichnet sind und wobei die Vorrichtung einen Lichtempfänger (120, 330, 440, 450, 530, 540), eine Vielzahl von Lichtsendern (110, 321, 322, 421, 422, 423, 521, 522, 523), die sich entsprechend einer Vielzahl von Farbsegmenten an einer Oberfläche des Biosensors (310, 410, 510) befinden und diesen zugewandt sind, wenn der Biosensor (310, 410, 510) in die Vorrichtung eingesetzt ist, und einen Identifikationscodeleser (130) umfasst, **dadurch gekennzeichnet, dass**
die Vielzahl von Lichtsendern (110, 321, 322, 421, 422, 423, 521, 522, 523) um den Lichtempfänger (120, 330, 440, 450, 530, 540) herum angeordnet ist;
und der Identifikationscodeleser (130) konfiguriert ist, um die Vielzahl von Lichtsendern (110, 321, 322, 421, 422, 423, 521, 522, 523) gemäß jeder Position davon sequenziell ein-/auszuschalten und den Lichtempfänger (120, 330. 440, 450, 530, 540) zu lesen, um Identifikationsinformationen zu lesen, die an dem Segment der Oberfläche des Biosensors aufgezeichnet sind, das einer Position des Lichtsenders (110, 321, 322, 421, 422, 423, 521, 522, 523), der eingeschaltet worden ist, entspricht und diesem zugewandt ist.

2. Vorrichtung nach Anspruch 1, wobei der Lichtempfänger (120, 330, 440, 450, 530, 540) eine Vielzahl von Fotodetektoren umfasst, die in Reihe oder parallel miteinander verbunden sind und die dazu in der Lage sind, von dem Identifikationscodeleser (130) gleichzeitig in ihrer Vollständigkeit simultan betrieben zu werden.

3. Vorrichtung nach Anspruch 1, wobei die Vielzahl von Lichtsendern (110, 321, 322, 421, 422, 423, 521, 522, 523) eine Vielzahl von Lichtsendeelementen umfassen, die simultan von dem Identifikationscodeleser (130) betrieben werden.

4. Vorrichtung nach Anspruch 3, wobei der Identifikationscodeleser (130) konfiguriert ist, um die Vielzahl von Lichtsendern (110, 321, 322, 421, 422, 423, 521, 522, 523) ein- und auszuschalten, indem zwischen Gruppen an Lichtsendern gewechselt wird, die als lineare Reihen entlang senkrechter Richtungen, die auf der Ebene des Biosensors (310, 410, 510) definiert sind, angeordnet sind.

5. Vorrichtung nach Anspruch 1, umfassend:
eine Vielzahl von Sätzen an Lesermodulen, enthaltend den Lichtempfänger (330), die Vielzahl von Lichtsendern (110, 321, 322, 421, 422, 423, 521, 522, 523) und den Identifikationscodeleser (130).

6. Vorrichtung nach Anspruch 3, wobei der Identifikationscodeleser (130) Folgendes umfasst:
einen Lichtsenderantrieb (210), der mit jedem aus der Vielzahl von Lichtsendern (110, 321, 322, 421, 422, 423, 521, 522, 523) verbunden ist;
einen Lichtempfängerantrieb (220), der konfiguriert ist, um den Lichtempfänger (120, 330, 440, 450, 530, 540) anzutreiben;
einen Umwandler (230), der konfiguriert ist, um eine Ausgabe des Lichtempfängers (120, 330, 440, 450, 530, 540) in ein digitales Format umzuwandeln; und
einen Musterleser (240), der konfiguriert ist, um ein Muster aus der Vielzahl von Farbsegmenten an einer Oberfläche des Biosensors (310, 410, 510) aus einer Ausgabe des Umwandlers (230) zu lesen.

7. Verfahren zum Lesen von Identifikationsinformationen eines Biosensors (310, 410, 510), wobei die Identifikationsinformationen gemäß einer Anordnung an unterschiedlichen Farben an einer Vielzahl von Segmenten an einer Oberfläche des Biosensors (310, 410, 510) aufgezeichnet sind, wobei das Verfahren Folgendes umfasst:
a) Erfassen eines Einsatzes des Biosensors (310, 410, 510);
b) Einschalten von einem aus einer Vielzahl von Lichtsendern (110, 321, 322, 421, 422, 423, 521, 522, 523), die in einer Position angeordnet sind, die Farbsegmenten an der Oberfläche des Biosensors (310, 410, 510), der eingesetzt worden ist, entsprechen und diesen zugewandt sind, während der Rest ausgeschaltet wird;
c) Erfassen, an einem Lichtempfänger (120, 330, 440, 450, 530, 540), eines Lichts, das von dem Lichtsender (110, 321, 322, 421, 422, 423, 521, 522, 523) projiziert worden ist und von dem Biosensor (310, 410, 510) reflektiert oder durch diesen penetriert worden ist; **dadurch gekennzeichnet, dass** das Verfahren weiter Folgendes umfasst:
d) Lesen, durch das erfasste Licht, einer Farbe, die in einer Position aufgezeichnet ist, die der Vielzahl von Farbsegmenten an der Oberfläche des Biosensors (310, 410, 510), der eingesetzt worden ist, entspricht; und
e) Lesen von Identifikationsinformationen durch Wiederholen der Vorgänge b) bis d) in Bezug auf die Vielzahl von Lichtsendern (110, 321, 322, 421, 422, 423, 521, 522, 523), die sich an unterschiedlichen Positionen befinden, und durch Lesen der Farben aller Segmente.

8. Verfahren nach Anspruch 7, wobei der Vorgang e) das Ein- und Ausschalten der Vielzahl von Lichtsendern (110, 321, 322, 421, 422, 423, 521, 522, 523) durch Wechseln zwischen Gruppen an Lichtsendern, die als lineare Reihen entlang senkrechter Richtungen, die auf der Ebene des Biosensors (310, 410, 510) definiert sind, angeordnet sind, umfasst.

## Revendications

1. Appareil configuré pour détecter une insertion d'un biocapteur et lire des informations d'identification du biocapteur (310, 410, 510), les informations d'identification étant enregistrées conformément à une configuration d'une pluralité de segments de couleur sur une surface du biocapteur, l'appareil comprenant
un récepteur lumineux (120, 330, 440, 450, 530, 540), une pluralité d'émetteurs de lumière (110, 321, 322, 421, 422, 423, 521, 522, 523), situés en correspondance avec une pluralité de segments de couleur sur une surface du biocapteur (310, 410, 510), le biocapteur (310, 410, 510) étant introduit dans l'appareil, et un lecteur de code d'identification (130),
**caractérisé en ce que**
la pluralité d'émetteurs de lumière (110, 321, 322, 421, 422, 423, 521, 522, 523) étant agencés autour du récepteur lumineux (120, 330, 440, 450, 530, 540) ;
et le lecteur de code d'identification (130) étant configuré pour mettre séquentiellement en/hors circuit la pluralité d'émetteurs de lumière (110, 321, 322, 421, 422, 423, 521, 522, 523) en fonction de la position de chacun, lire le récepteur lumineux (120, 330, 440, 450, 530, 540) pour relever les informations d'identification enregistrées sur le segment de la surface du biocapteur correspondant à une position de l'émetteur de lumière (110, 321, 322, 421, 422, 423, 521, 522, 523) qui a été mis en circuit, et tourné vers celle-ci.

2. Appareil selon la revendication 1, le récepteur lumineux (120, 330, 440, 450, 530, 540) comprenant une pluralité de photo-détecteurs raccordés en série, ou parallèles entre eux, et pouvant être utilisés simultanément dans leur intégralité par le lecteur de code d'identification (130).

3. Appareil selon la revendication 1, la pluralité d'émetteurs de lumière (110, 321, 322, 421, 422, 423, 521, 522, 523) comprenant une pluralité d'éléments électroluminescents actionnés simultanément par le lecteur de code d'identification (130).

4. Appareil selon la revendication 3, le lecteur de code d'identification (130) étant configuré pour mettre en/hors circuit la pluralité d'émetteurs de lumière (110, 321, 322, 421, 422, 423, 521, 522, 523) en alternant entre des groupes d'émetteurs de lumière agencés sous forme de réseaux linéaires le long de directions perpendiculaires définies dans le plan du biocapteur (310, 410, 510).

5. Appareil selon la revendication 1, comprenant :
une pluralité d'ensembles de modules de lecteur comprenant le récepteur lumineux (330), la pluralité d'émetteurs de lumière (110, 321, 322, 421, 422, 423, 521, 522, 523), et le lecteur de code d'identification (130).

6. Appareil selon la revendication 3, le lecteur de code d'identification (130) comprenant :
un pilote d'émetteur de lumière (210) connecté à chacun de la pluralité des émetteurs de lumière (110, 321, 322, 421, 422, 423, 521, 522, 523) ;
un pilote de récepteur lumineux (220) configuré pour commander le récepteur lumineux (120, 330, 440, 450, 530, 540) ;
un convertisseur (230) configuré pour convertir une sortie du récepteur lumineux (120, 330, 440, 450, 530, 540) dans un format numérique ; et
un lecteur de configuration (240) configuré pour relever une configuration dans une pluralité de segments de couleur sur une surface du biocapteur (310, 410, 510) d'après une sortie du convertisseur (230).

7. Méthode de lecture d'informations d'identification d'un biocapteur (310, 410, 510), les informations d'identification étant enregistrées conformément à un agencement de différentes couleurs sur une pluralité de segments sur une surface du biocapteur (310, 410, 510), la méthode comprenant :
a) la détection d'une introduction du biocapteur (310, 410, 510) ;
b) la mise en circuit d'un d'une pluralité d'émetteurs de lumière (110, 321, 322, 421, 422, 423, 521, 522, 523) agencés dans une position correspondant à des segments de couleur sur la surface du biocapteur (310, 410, 510) qui a été introduit, et leur faisant face, tout en mettant le restant hors circuit ;
c) détection, sur un récepteur lumineux (120, 330, 440, 450, 530, 540), d'une lumière qui a été projetée par l'émetteur de lumière (110, 321, 322, 421, 422, 423, 521, 522, 523) et reflétée par le biocapteur (310, 410, 510) ou ayant pénétré dans celui-ci ;
**caractérisée en ce que** la méthode comprend en outre :
d) la lecture, à travers la lumière détectée, d'une couleur enregistrée dans une position correspondant à la pluralité de segments de couleur sur la surface du biocapteur (310, 410, 510) que l'on a introduit ; et
e) la lecture d'informations d'identification en répétant les opérations b) à d) relativement à la pluralité d'émetteurs de lumière (110, 321, 322, 421, 422, 423, 521, 522, 523) situés dans différentes positions, et en relevant les couleurs de tous les segments.

8. Méthode selon la revendication 7, l'opération e) comprenant la mise en et hors circuit de la pluralité d'émetteurs de lumière (110, 321, 322, 421, 422, 423, 521, 522, 523) en alternant entre les groupes d'émetteurs de lumière agencés sous forme de réseaux linéaires dans des directions perpendiculaires définies dans le plan du biocapteur (310, 410, 510).
